# EUROPEAN PATENT APPLICATION

(11) **EP 2 087 837 A1**
(43) Date of publication of application: **12.08.2009**
(21) Application number: 06833399.6
(22) Date of filing: 27.11.2006
(51) Int. Cl.: A61B 5/026, A61B 5/0285, A61B 5/1455, G01N 21/35

(54) **EMISSION SENSOR DEVICE AND BIOINFORMATION DETECTING METHOD**

(71) Applicant: Pioneer Corporation, Tokyo 153-8654 (JP); Kyushu University, Higashi-ku Fukuoka-shi Fukuoka 812-8581 (JP)
(72) Inventor: KIMURA, Yoshinori, Tsurugashima-shi Saitama 350-2288 (JP); ONOE, Atsushi, Tsurugashima-shi Saitama 350-2288 (JP); SAWADA, Renshi, Fukuoka-shi Fukuoka 812-8581 (JP)
(74) Representative: Viering, Jentschura & Partner
(86) International application number: PCT/JP2006/323595
(87) International publication number: WO 2008/065699

(57) **Abstract**

An emission sensor device comprises a base (110), a light applying section (120) disposed on the base and adapted for applying light beams having different wavelengths to a subject in such a way that the light beams at least overlap with one another, and a light receiving section (150) disposed on the base and adapted for detecting light from the subject attributed to the applied light beams for each wavelength. The device has a small size and can collect predetermined types of information on the subject such as bioinformation with high accuracy

## Description

### Technical Field

The present invention relates to, for example, a light-emitting sensor apparatus which can measure a blood flow velocity and the like, and a method of inspecting biological information using the light-emitting sensor apparatus.

### Background Art

As this type of light-emitting sensor apparatus, there is an apparatus for applying light, such as a laser beam, to an object and for calculating a velocity or the like of the object from a change in wavelength caused by Doppler shift in reflection or scattering of the light. For example, there has been suggested a blood flow measuring apparatus which can measure a blood flow for blood vessels located in different depths by applying laser beams with different wavelengths from respective two laser diodes (refer to a patent document 1).

Patent document 1: Japanese Patent Application Laid Open No. 2004-229920

### Disclosure of Invention

### Subject to be Solved by the Invention

However, according to the technology as described above, in order to make a measurement by the light with the different wavelengths, at least two laser elements are to be provided, and this causes an increase in size of the apparatus, which is technically problematic. Moreover, since the laser beams with the different wavelengths are emitted from different places, it is extremely hard to make a measurement at the same site, which is also technically problematic.

In view of the aforementioned problems, it is therefore an object of the present invention to provide a light-emitting sensor apparatus which is suitable for miniaturization and which can highly accurately detect a predetermined type of information in a measured sample, such as biological information, and a detection method which can detect the biological information, highly accurately, using the light-emitting sensor apparatus.

### Means for Solving the Subject

The above object of the present invention can be achieved by a light-emitting sensor apparatus provided with: a substrate; an applying device which is disposed on the substrate and which applies a plurality of light with mutually different wavelengths to a measured sample such that the light overlaps each other at least partially; and a light receiving device which is disposed on the substrate and which detects light from the measured sample caused by the applied plurality of light, for each wavelength.

According to the light-emitting sensor apparatus of the present invention, in the detection, the plurality of light with the mutually different wavelengths is applied to the measured sample, which is, for example, one portion of a biological object, by the applying device including a semiconductor laser. Moreover, the plurality of light is applied to the measured sample such that the light overlaps each other at least partially (hereinafter referred to "application to the same site", as occasion demands). Incidentally, the same site can denote points located in mutually different depths, in terms of a depth direction of the measured sample. Moreover, the expression that "... overlaps each other at least partially" means that there is a portion in which the plurality of light overlaps each other, to a greater or lesser degree, on the surface of the measured sample irradiated with the light.

The plurality of light is sequentially or simultaneously applied from, for example, a single semiconductor laser. Here, if the light with the different wavelengths is applied from two semiconductor lasers, the layout area is twice or more in simple calculation. In contrast, if the semiconductor lasers are arranged in a narrower area than usual or closer to each other in order to prevent the increase in layout area, there is more difficulty in chip implementation, wiring, and the like, which problematically causes a reduction in yield.

In the present invention, however, in particular, the plurality of light with the different wavelengths is applied from the applying device, for example, such as a single semiconductor laser device having light-emitting devices close to each other. Thus, the layout area can be significantly reduced. This facilitates the miniaturization of the light-emitting sensor apparatus and the application of the light with the different wavelengths to the same site. In particular, since a plurality of light-emitting points of the plurality of light with the different wavelengths are close to each other and disposed in the single semiconductor laser device, there is less difficulty in chip implementation, wiring, and the like, which can improve a production yield. Moreover, that facilitates the application of the light to the same site of the measured sample, for example, through the same optical system, such as a single condenser lens.

Moreover, the light from the measured sample caused by the plurality of light applied in this manner is detected by the light receiving device including, for example, a light receiving element. Here, the "light from the measured sample caused by the plurality of light" denotes light caused by the plurality of light applied to the measured sample, such as light reflected, scattered, diffracted, refracted, transmitted, and Doppler-shifted, and their interfering light. Typically, it may be the reflected and scattered light, or Doppler-shifted light, and their interfering light. The light caused by the plurality of light with the different wavelengths can be detected or divided, sequentially or in a simultaneous parallel process, by a method in which wavelength division is performed by a time-division process after the plurality of light with the different wavelengths is alternately emitted by time division and is received by a single light receiving device, or by a method in which wavelength division is performed by arranging a plurality of exclusive light receiving devices corresponding to the respective wavelengths, or by similar methods. Thus, it is possible to obtain information such as biological information, with each of the light with different wavelengths. For example, it is also possible to derive new information by comparing information obtained by emitting light with one wavelength and information obtained by emitting light with another wavelength.

Here in the present invention, in particular, the applying device and the light receiving device are arranged on the same substrate, and, for example, they can be also disposed close to each other, as an integrated circuit. By arranging the applying device and the light receiving device on the same substrate, the layout area is reduced for each of the applying device and the light receiving device. Therefore, the entire apparatus can be miniaturized.

As described above, according to the present invention, it is possible to miniaturize the light-emitting sensor apparatus, and moreover, it is possible to detect a plurality type of information on the measured sample, such as biological information, highly accurately.

In one aspect of the light-emitting sensor apparatus of the present invention, the applying device applies the plurality of light through a common optical system.

According to this aspect, the light emitted from the applying device is applied to the measured sample, for example, through one microlens. The microlens makes the irradiated light parallel, to thereby function to keep a constant energy density of the irradiated light on the surface of the measured sample, regardless of a distance between the measured sample and the microlens.

Here, if a plurality of microlenses are arranged, the lens is to have a physically small diameter due to a limit in space for the arrangement. The small lens diameter causes a reduction in intensity of the irradiated light or a reduction in use efficiency of the light emitted from the laser element (or efficiency of binding to the lens), resulting in a reduced SNR (Signal to Noise Ratio) and increased power consumption.

In the present invention, however, in particular, the light-emitting points of the light with the different wavelengths are close to each other and are located in one laser element, so that it is easy to establish the construction that one common microlens may be provided for the light with the different wavelengths. Thus, it is possible to increase the lens diameter of the microlens, and it is possible to increase the intensity of the irradiation light and the use efficiency of the light emitted from the laser element. Therefore, it is possible to realize the light-emitting sensor apparatus which provides a better SNR and lower power consumption.

In another aspect of the light-emitting sensor apparatus of the present invention, the applying device sequentially applies the plurality of light, and the light receiving deice detects the light from the measured sample for each wavelength by time division.

According to this aspect, the light with the different wavelengths is sequentially applied from the applying device and is not simultaneously applied. In other words, at a time point at which light with one wavelength is applied, light with another wavelength is not applied. Thus, even in the case of one light receiving device, it is easy to detect the light for each wavelength by time-division. Therefore, a plurality of light receiving elements are not necessarily provided, which provides effective miniaturization of the sensor. Moreover, since the laser beams with the different wavelengths are sequentially driven, it is possible to set a low drive current value per time, which allows lower power consumption.

In another aspect of the light-emitting sensor apparatus of the present invention, the applying device simultaneously applies the plurality of light, and the light receiving deice has a plurality of light receiving elements which detect the light from the measured sample for each wavelength.

According to this aspect, the light with the different wavelengths is simultaneously applied from the applying device, so that it is possible to obtain information at the same time point from each light. Thus, it is possible to make an accurate measurement if a parameter which changes in an extremely short time is measured or in similar cases. Moreover, since a process is simultaneously performed on the information obtained from the light with the different wavelengths, it is possible to reduce a time for the measurement.

Incidentally, it is certain on the light receiving device that the detection for each wavelength can be performed by providing a plurality of light receiving elements corresponding to the respective wavelengths in the irradiation.

In another aspect of the light-emitting sensor apparatus of the present invention, the applying device and the light receiving device are integrated on the substrate.

According to this aspect, since the applying device and the light receiving device are integrated, it is possible to reduce the layout area for each of them and to miniaturize the apparatus. The miniaturization can expand the application range, such as providing the apparatus not of a stationary type but of a portable type.

In another aspect of the light-emitting sensor apparatus of the present invention, the applying device has a semiconductor laser which generates a plurality of laser beams as the plurality of light.

According to this aspect, by applying a voltage to the semiconductor laser of the applying device such that a higher electric current than a laser oscillation threshold value is flown thereto, it is possible to apply the laser beam. The laser beam is **characterized in that** the penetration force to a biological object or the like varies depending on the difference in wavelength. By using this characteristic, it is possible to make a measurement in various depths of the measured sample.

In an aspect in which the applying device has the semiconductor laser, as described above, the semiconductor laser may make a drive current lower than a laser oscillation threshold value, to thereby function as a light-emitting diode.

By virtue of such construction, the semiconductor laser functions as the light-emitting diode (LED), and the irradiated laser is not coherent light like the laser beam. Thus, for example, it is not suitable for a measurement using a change in wavelength by the Doppler shift of the light caused by the transfer rate of red blood cells in blood vessels. However, it is possible to make a measurement using a change in intensity of the light caused by the optical absorption of red blood cells, or the like. For example, since an optical absorption factor of the measurement sample is obtained from the intensity of the reflected or scattered light, the measurement can be made even if the semiconductor laser functions as the light-emitting diode. Thus, by combining and using the plurality of light with the different wavelengths, as occasion demands, it is possible to measure the concentration of oxygen contained in blood.

In the case where the semiconductor laser functions as the light-emitting diode, a driving power is lower than in the case where the laser beam is applied. Therefore, it is possible to reduce power consumption by controlling the drive current of the semiconductor laser depending on the measurement target.

In another aspect of the light-emitting sensor apparatus of the present invention, the applying device is provided with: a light source for generating a plurality of source light; and a reflecting mirror which reflects the generated plurality of source light in a direction of the measured sample so as to be the irradiated plurality of light, at a position irradiated with the generated plurality of source light.

According to this aspect, for example, it is possible to direct the travelling direction of the light applied from the light source, such as a semiconductor laser, in the direction of the measured sample (or toward the measured sample) by reflecting the light with the reflecting mirror disposed at a proper angle in advance. Here, the "direction" in the direction of the measured sample denotes a so-called direction in a broad sense; for example, not only a direction directly going to the measured sample but also a direction to the measured sample through an optical system such as a lens and another mirror. Moreover, since the reflecting mirror having high reflectance for both the different wavelengths is used, the intensity of the light emitted from the laser element is maintained before and after the reflection by the reflecting mirror, and the light is applied to the measured sample by the microlens, with a proper power density kept. Thus, the detection can be correctly performed on the light receiving device. Incidentally, in the present invention, since the light with the different wavelengths is applied from the light-emitting points close to each other, one reflecting mirror can reflect both the light with the different wavelengths.

In an aspect further provided with the reflecting mirror, as described above, the reflecting mirror may include, on its surface, at least one of Au (gold), Cu (copper), Al (aluminum), and Ag (silver).

In this case, since the reflecting mirror includes, on its surface, at least one of Au, Cu, Al, and Ag which have high reflectance, it is possible to apply the light with the different wavelengths emitted from the applying device, to the measured sample, efficiently. Moreover, each of Au, Cu, Al, and Ag has high reflectance in a different range of wavelength, so that by using any of the metal or by combining the plurality of metal in accordance with the applied light with the plurality of different wavelengths, it is possible to set the reflecting mirror to have high reflectance with respect to the respective wavelengths. Moreover, in addition to the aforementioned metal, it is also possible to form a dielectric multilayer film in a lamination structure of SiO₂, TiO₂, or the like on the surface of the reflecting mirror and to form the reflecting mirror having high reflectance.

In another aspect of the light-emitting sensor apparatus of the present invention, it is further provided with a calculating device for calculating at least one of a blood flow velocity and a blood oxygen saturation level associated with the measured sample on the basis of the detected light.

According to this aspect, it is possible to measure a blood flow velocity for blood vessels located in different depths from the skin surface, by using that the penetration force of the light to a biological object depends on the wavelength. Specifically, by applying the light to the surface of the biological object, the light passes through the skin is reflected or scattered by red blood cells flowing in blood vessels, and the wavelength is changed by the Doppler shift according to the transfer rate of red blood cells. On the other hand, the light reflected or scattered by a skin tissue or the like, which can be considered to be immobile with respect to the red blood cells, reaches to the light receiving device without the wavelength changed. By the light interfering with each other, an optical beat signal corresponding to a Doppler shift amount is detected on the light receiving device. An arithmetic process or the like, such as frequency analysis, is performed on the optical beat signal on the calculating device, and by this, the blood flow velocity flowing in blood vessels can be obtained.

In the present invention, in particular, since the plurality of light with the different wavelengths is used as the irradiated light, it is possible to measure the blood flow velocity for blood vessels located in depths corresponding to the respective wavelength. By measuring the blood flow velocity for blood vessels located in different depths, it is possible to obtain a blood flow velocity distribution in the depth direction. By obtaining the blood flow velocity distribution, for example, it is possible to predict a psychological status, such as the degree of tension, from a difference in the blood flow velocity between blood capillaries located near the skin and an arteriole located deeper than the blood capillary.

Moreover, it is possible to measure the blood oxygen saturation level by using a difference in absorbance between oxygenated hemoglobin and reduced hemoglobin, and the dependency on wavelength. Specifically, the plurality of light with the different wavelengths is applied to the same site of the measured sample, and the intensity of reflected light is measured for each of the light. At this time, the light is absorbed and reflected by hemoglobin contained in blood of the measured sample, and the absorption factor varies depending on whether the hemoglobin is oxygenated hemoglobin or reduced hemoglobin. Thus, the intensity of reflected light varies depending on an oxygenated hemoglobin ratio (i.e. blood oxygen saturation level). Moreover, the absorbance of hemoglobin depends on the wavelength of light, and the absorbance of each of oxygenated hemoglobin and reduced hemoglobin indicates different characteristics depending on the wavelength of the irradiated light. Thus, the reflected light of each of the plurality of light with the different wavelengths simultaneously applied has different intensity. Thus, it is possible to calculate the blood oxygen saturation level by using the plurality of light with the different wavelengths.

Incidentally, in the present invention, in particular, it is possible to simultaneously measure the blood flow velocity and the blood oxygen saturation level described above. By simultaneously measuring the blood flow velocity and the blood oxygen saturation level, it is possible to eliminate a signal component which is changed by breathing, obtained by the blood oxygen saturation level measurement, from a signal obtained by the blood flow velocity measurement, and this allows more accurate detection of the change in blood flow velocity caused by the psychological status of the measured sample.

The above object of the present invention can be achieved by a method of detecting biological information using a light-emitting sensor apparatus provided with: an applying device on a substrate; and a light receiving device, the method provided with: an applying process of applying a plurality of light with mutually different wavelengths to a measured sample such that the light overlaps each other at least partially; and a light receiving process of detecting light from the measured sample caused by the applied plurality of light, for each wavelength.

According to the method of detecting biological information in the present invention, for example, since the plurality of light with the different wavelengths is applied from one laser element, it is possible to detect the biological information using the light with the different wavelengths, without complicating the light emitting process and the light receiving process. Moreover, it facilitates the application to the same site of the light with the different wavelengths with respect to the measured sample, which allows extremely accurate detection of the biological information.

The operation and other advantages of the present invention will become more apparent from Best Mode for Carrying Out the Invention explained below.

### Brief Description of Drawings

[FIG. 1] FIG. 1 is a plan view showing the structure of a sensor device in a two-wavelength blood flow sensor apparatus in embodiments of the present invention.
[FIG. 2] FIG. 2 is an A-A' cross sectional view in FIG. 1.
[FIG. 3] FIG. 3 is a top view showing the structure of a hybrid two-wavelength laser by bonding.
[FIG. 4] FIG. 4 is a top view showing the structure of a monolithic two-wavelength laser.
[FIG. 5] FIG. 5 is a conceptual view showing an effective working distance of the two-wavelength blood flow sensor apparatus.
[FIG 6] FIG. 6 is a block diagram showing the structure of a two-wavelength blood flow sensor apparatus in the first embodiment.
[FIG. 7] FIG. 7 is a conceptual view showing one example of how to use the two-wavelength blood flow sensor apparatus.
[FIG. 8] FIG. 8 is a graph showing a relation between absorbance and wavelength in hemoglobin.
[FIG. 9] FIG. 9 is a cross sectional view showing a human subcutaneous structure.
[FIG 10] FIG. 10 is a block diagram showing the structure of a two-wavelength blood flow sensor apparatus in the second embodiment.

### Description of Reference Codes

100, 200 sensor device
110 sensor device substrate
120, 121, 122 two-wavelength laser diode
130 reflecting mirror
140 microlens
150 photo diode
210 laser diode drive circuit
220 first switching circuit
230 photo diode amplifier
240 A/D converter
250 second switching circuit
260 first blood flow DSP
270 second blood flow DSP
280 autonomic function evaluation DSP
290 blood oxygen saturation level DSP
410 arteriole
420 venula
430 blood capillary
440 precapillary sphincter

### Best Mode for Carrying Out the Invention

Hereinafter, embodiments of the present invention will be described with reference to the drawings. In the following embodiments, a two-wavelength blood flow sensor apparatus which is one example of the light-emitting sensor apparatus of the present invention is taken as an example.

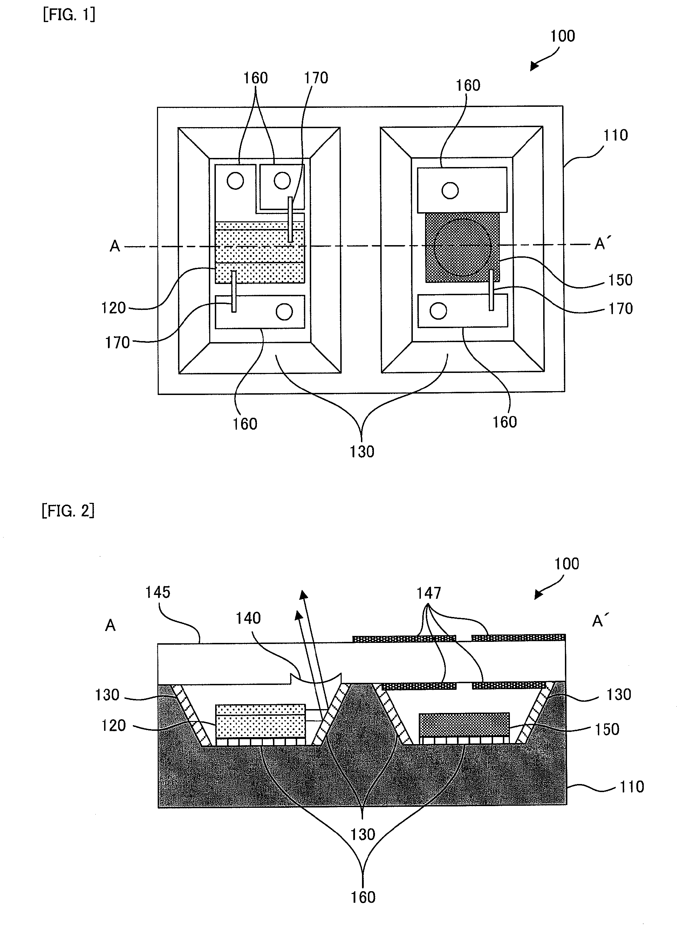

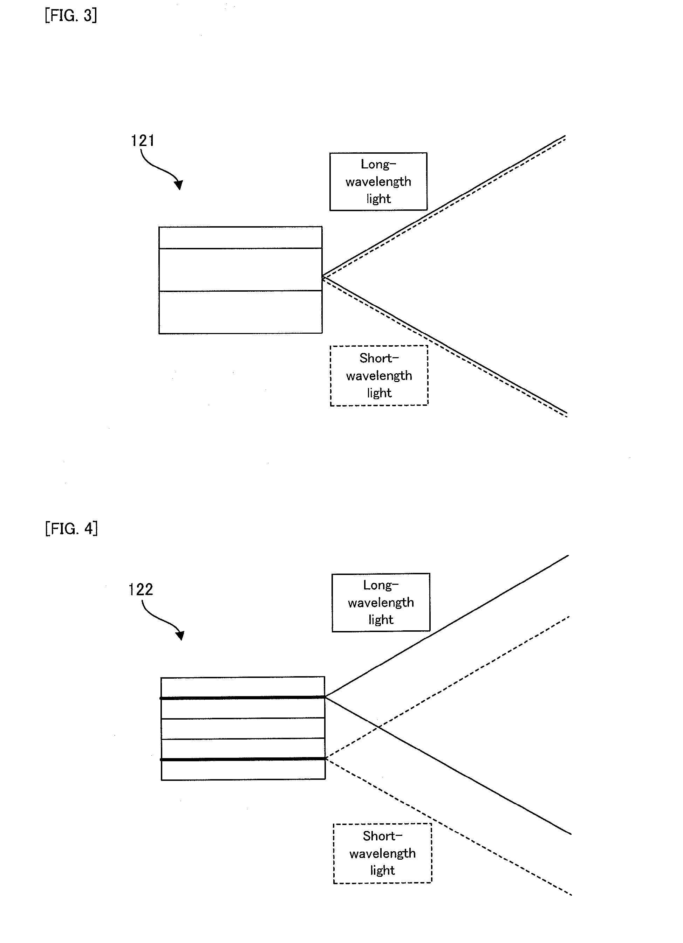

Firstly, the structure of a two-wavelength blood flow sensor apparatus in the first embodiment of the present invention will be described with reference to FIG. 1 to FIG. 4. FIG. 1 is a plan view showing the structure of a sensor device in the two-wavelength blood flow sensor apparatus. FIG. 2 is an A-A' cross sectional view in FIG. 1. FIG. 3 is a top view showing the structure of a hybrid two-wavelength laser by bonding. FIG. 4 is a top view showing the structure of a monolithic two-wavelength laser. Incidentally, in FIG. 1, for convenience of explanation, an illustration of a transparent substrate having a microlens is omitted.

As shown in FIG. 1 and FIG. 2, a sensor device 100 of the two-wavelength blood flow sensor apparatus in the first embodiment is provided with a sensor device substrate 110, a two-wavelength laser diode 120, reflecting mirrors 130, a transparent substrate 145 having a microlens 140, shielding films 147, a photo diode 150, electrodes 160, and wirings 170

On the sensor device substrate 110, the two-wavelength laser diode 120 and the photo diode 150 are integrated and disposed, and each is electrically connected to respective one of the electrodes 160 through the wirings 170. Moreover, each of the electrodes 160 is electrically connected to an electrode pad (not illustrated) disposed at the bottom of the sensor device substrate 110 by a wiring (not illustrated) passing through the sensor device substrate 110. Thus, it is possible to drive the two-wavelength laser diode 120 by current injection from the exterior of the sensor device and to extract a signal from the photo diode 150 to the exterior of the sensor.

As shown in FIG. 3, the two-wavelength laser diode 120 is, for example, a hybrid two-wavelength laser 121 by bonding. Specifically, it is a single laser diode chip by bonding two semiconductor lasers, which are prepared on different substrates and which emit laser beams with mutually different wavelengths, to each other at a stage of laser wafer or laser diode chip. According to the hybrid two-wavelength laser 121 by bonding, since it has a high degree of freedom in combination of the wavelengths of the emitted light, it is possible to realize the two-wavelength laser in various combinations, regardless of the substrates. Moreover, since the semiconductor lasers are integrated by bonding, light emitting points can be placed next to each other in a direction perpendicular to the substrates on which the semiconductor lasers are disposed. Thus, it is possible to place two laser emitting points close enough to be at an interval of about several µm. Placing the light emitting points close to each other, as shown in FIG. 3, allows the laser beams with different wavelengths (long-wavelength light and short-wavelength light) to be emitted in the same direction from extremely close places. This provides a beneficial effect when light is focused by an optical system such as the microlens 140; namely, it is possible to focus the laser beams from the two light emitting points with a common lens, and it is also easy to apply the laser beams to the same site of a measured sample.

As shown in FIG. 4, the two-wavelength laser diode 120 may be, for example, a monolithic two-wavelength laser 122 in which two semiconductor lasers are prepared on one substrate. However, the monolithic two-wavelength laser 122 has a lower degree of freedom in combination of the wavelengths of the emitted light, compared to the aforementioned hybrid two-wavelength laser 121 by bonding. Moreover, since the two laser devices are disposed on the same substrate, the both semiconductor lasers are arranged in a direction along the substrate. Thus, the interval between the two laser emitting points is increased (typically, about several hundreds µm), and the portion in which the long-wavelength light and the short-wavelength light overlap is narrower than in the case of the hybrid two-wavelength laser 121 by bonding. Therefore, placing the light emitting points close to each other, as described above, has less effect, so that the hybrid two-wavelength laser 121 by bonding is more desirably used than the monolithic two-wavelength laser 122 in the embodiment.

For the semiconductor laser of the two-wavelength laser diode 120, for example, a relatively inexpensive Fabry-Perot (FP) laser is used in most cases. However, the FP laser provides longitudinal multimode oscillation, so that there are many laser noises (i.e. laser beam output variations) caused by mode hopping or the like, and this likely reduces the measurement accuracy. With respect to this problem, the laser noises can be reduced by providing a high-frequency superimposing circuit to superimpose a high frequency. Moreover, instead of the high-frequency superimposing circuit, a self-oscillation laser (or pulsation laser) can be also used to reduce an influence of the laser noises. Moreover, not the FP laser but a distributed-feedback (DFB) laser which provides dynamic longitudinal single mode oscillation can be also used to reduce the laser noises.

Incidentally, in the embodiment, the two-wavelength laser diode 120 is used as one example of the "applying device"; however, a laser diode which can emit laser beams with two wavelengths or more, i.e. three wavelengths and four wavelengths, can be also used to obtain much more data in a single measurement.

The reflecting mirrors 130 are disposed at positions directly irradiated with the laser beams with the different wavelengths from the two-wavelength laser diode 120, and each of the reflecting mirrors 130 reflects the laser beam toward the measured sample. The reflected laser beams have different wavelengths, so that the reflecting mirrors 130 preferably have high reflectance for the both wavelengths. For example, by coating the surface with Au, Cu, Al, or the like, it is possible to obtain the reflecting mirror having high reflectance for light with a wavelength of 600 nm to 1500 nm. Moreover, in using a short-wavelength laser beam with a wavelength of about 400 nm, Al, Ag, or the like is preferably used which has high reflectance in a wider wavelength range. However, since Al and Ag are easily oxidizable, some oxidation preventing measure is preferably taken, such as air-tight sealing.

Moreover, by combining a plurality of materials, it is also possible to make the reflecting mirrors 130 have high reflectance in a wider range of wavelength. Incidentally, if the light from the two-wavelength laser diode 120 is directly emitted toward the measured sample, the reflecting mirrors 130 are not necessarily provided.

The microlens 140 is disposed, for example, above the two-wavelength laser diode 120 as one portion of the transparent substrate 145, as shown in FIG. 2, to make the incident laser beam parallel and increase intensity and laser-beam use efficiency. As described above, the light emitting points of the laser beams are close to each other in the present invention, so that it is only necessary to provide one microlens 140 for the two light emitting points. Thus, it is possible to increase the diameter of the microlens 140, which allows the laser-beam use efficiency (or efficiency of binding to the lens) to be improved.

The photo diode 150 is arranged on the same substrate as that for the two-wavelength laser diode 120, and it detects the light reflected or scattered from the measured sample. Specifically, the photo diode 150 can obtain information about the light intensity by converting the light to an electric signal. Incidentally, on the transparent substrate 145 above the photo diode 150, there are provided a plurality of shielding films 147 which limit the light entering the photo diode 150. Thus, only the light from directly above (i.e. in a top-to-bottom direction in FIG. 2) enters the photo diode 150. Thus, it is possible to prevent the light that is not necessarily detected, from entering the photo diode 150, to thereby increase the detection accuracy. In the two-wavelength blood flow sensor apparatus in the embodiment, the light with the different wavelengths is sequentially emitted, so that the light can be divided for each wavelength by time division or time sharing. Thus, it is only necessary to provide one photo diode, which allows the miniaturization of the apparatus.

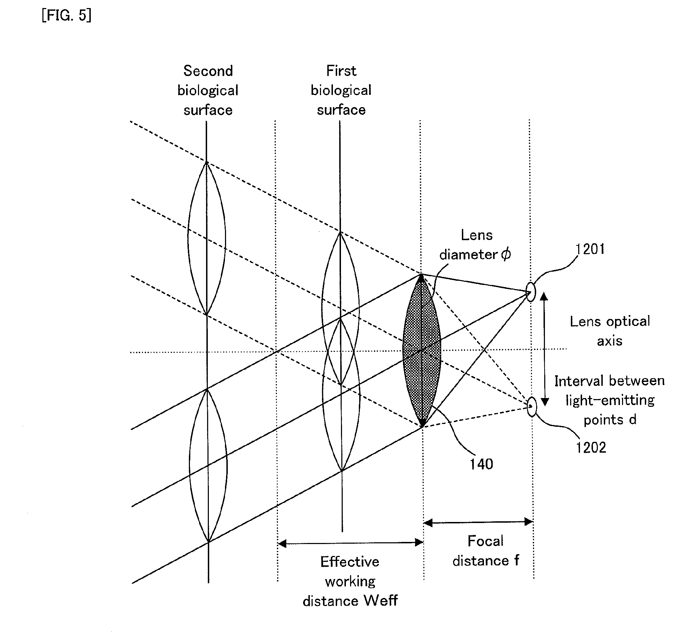

Next, an effective working distance of the two-wavelength blood flow sensor apparatus in the embodiment will be described with reference to FIG. 5. FIG. 5 is a conceptual view showing the effective working distance of the two-wavelength blood flow sensor apparatus.

As shown in FIG. 5, if the lens diameter of the microlens 140 is φ, a focal distance is f, an interval between the light emitting points 1201 and 1202 of the two-wavelength laser diode is d, and the effective working distance (i.e. a distance which allows the light with the different wavelengths to be applied to the same site) of the apparatus is Weff, then, a relation of Weff <φf / d is established among them. Thus, as the lens diameter φ increases, and as the light emitting point interval d reduces, the effective working distance Weff increases. Thus, in the present invention in which the microlens 140 can be expanded and in which the light emitting point interval d can be reduced, it is possible to increase the effective working distance and to expand the application range of the present invention.

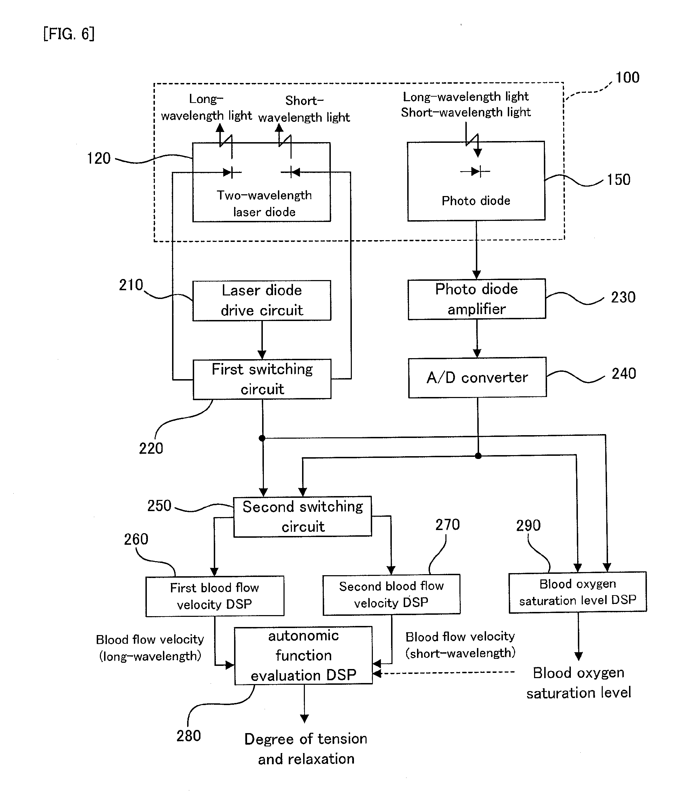

Next, the structure of the entire light-emitting sensor apparatus in the embodiment and its operational flow will be described with reference to FIG. 6. FIG. 6 is a block diagram showing the structure of a two-wavelength blood flow sensor apparatus in the first embodiment.

As shown in FIG. 6, the two-wavelength blood flow sensor apparatus in the embodiment is provided with a laser diode drive circuit 210, a first switching circuit 220, a photo diode amplifier 230, an A/D (Analog to Digital) converter 204, a second switching circuit 250, a first blood flow velocity DSP (Digital Signal Processor) 260, a second blood flow velocity DSP 270, an autonomic function evaluation DSP 280, and a blood oxygen saturation level DSP 290, in addition to the aforementioned sensor device 100.

Firstly, by a signal from the laser diode drive circuit 210 allowing the first switching circuit 220 to switch over and output the emission of the long-wavelength light (e.g. wavelength of 830 nm) and the emission of the short-wavelength light (e.g. wavelength of 780 nm), the light with the different wavelengths is sequentially emitted from the two-wavelength laser diode 120. The emitted light is reflected or scattered by the measured sample, and the reflected or scattered light is detected by the photo diode 150. Incidentally, in the embodiment, since the light with the different wavelengths is sequentially emitted, a drive current is lower than in a case where the light with the different wavelengths is simultaneously emitted, which provides lower power consumption. Moreover, since the plurality of light with the different wavelengths is not emitted at the same time point, the plurality of light can be divided by the common photo diode 150 by time division.

The electric signal obtained by the photo diode 150 is amplified by the photo diode amplifier 230 and then converted by the A/D converter 240 to a digital signal. The digital signal is time-divided into a signal by the long-wavelength light and a signal by the short-wavelength light, by using information about an emission time point of the light from the first switching circuit 220. In particular, in the calculation of a blood flow velocity, the signal is divided in advance by the second switching circuit 250 and then sent to each of the first blood flow velocity DSP 260 and the second blood flow velocity DSP 270. Incidentally, in order to omit the dividing operation by the second switching circuit 250, a plurality of photo diodes may be provided for respective wavelengths.

In the first blood flow velocity DSP 260 and the second blood flow velocity DSP 270, a blood flow velocity by the long-wavelength light (i.e. of a blood vessel located in greater depth) and a blood flow velocity by the short-wavelength light (i.e. of a blood vessel located in less depth) are calculated by performing a predetermined arithmetic process. The calculated blood flow velocities are sent to the autonomic function evaluation DSP 280 where a comparison arithmetic process is performed, providing data which indicates a psychological status, such as the degree of tension and relaxation, of the measured sample. On the one hand, in the blood oxygen saturation level DSP 290, a blood oxygen saturation level is calculated. Incidentally, in the autonomic function evaluation DSP 280, it is possible to make a more accurate measurement by using the blood oxygen saturation level calculated on the blood oxygen saturation level DSP 290 in addition to the blood flow velocities in different depths.

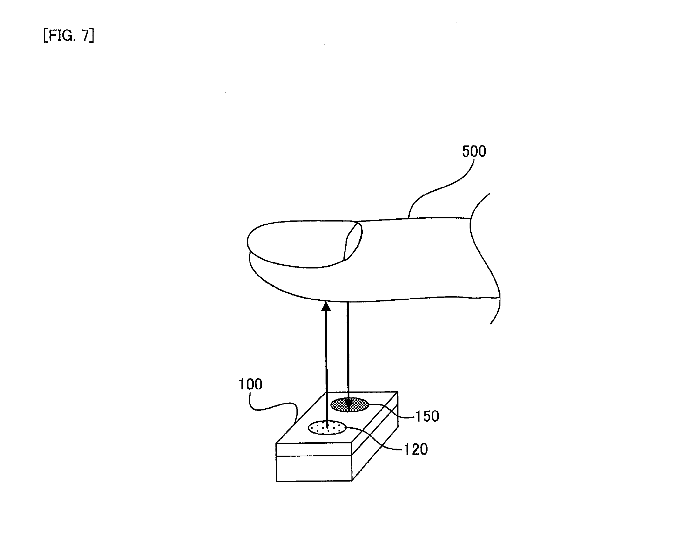

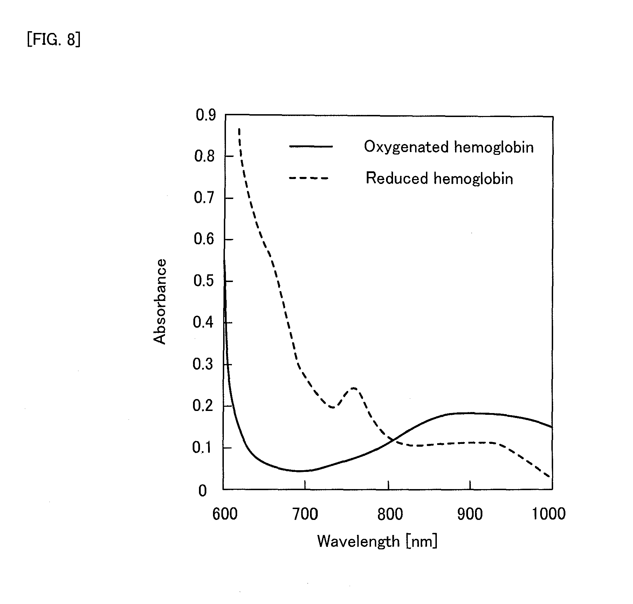

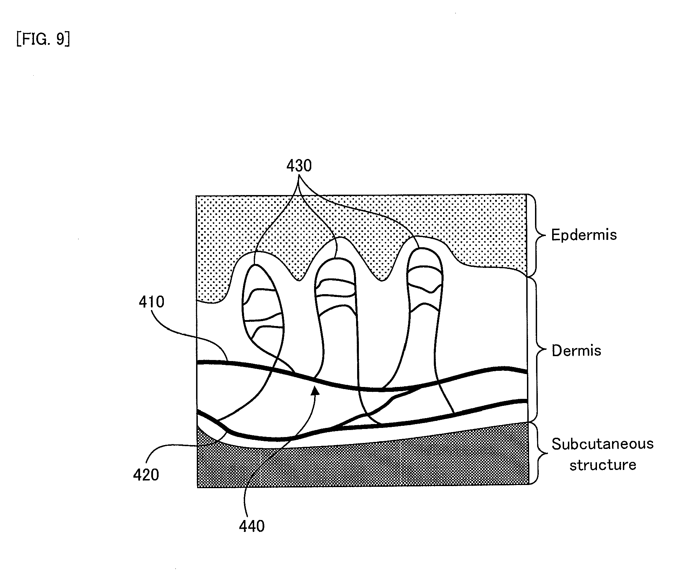

Next, the details and effect of the measurement of the blood flow velocity and the blood oxygen saturation level by the two-wavelength blood flow sensor apparatus in the embodiment will be described with reference to FIG. 7 to FIG. 9. FIG. 7 is a conceptual view showing one example of how to use the two-wavelength blood flow sensor apparatus. FIG. 8 is a graph showing a relation between absorbance and wavelength in hemoglobin. FIG. 9 is a cross sectional view showing a human subcutaneous structure.

As shown in FIG. 7, the two-wavelength blood flow sensor apparatus in the embodiment measures the blood flow velocity by applying a laser beam to a finger tip 500 which is a measured sample, using the two-wavelength laser diode 120. At this time, the portion irradiated with the laser beam is more desirably a portion in which blood capillaries are densely distributed at a relatively close position from the epidermis (e.g. a hand, a leg, a face, an ear, and the like). The laser beam applied to the finger tip 500 passes through the skin to different depths depending on the wavelength and is reflected or scattered by body tissues, such as blood and skin cells, of the measured sample. Moreover, red blood cells flowing in blood vessels cause the Doppler shift in the reflected or scattered light, which changes the wavelength of the light depending on the transfer rate of red blood cells, i.e. the blood flow velocity. On the one hand, the wavelength is not changed in the light reflected or scattered by the skin cells which can be considered to be immobile with respect to the red blood cells. By the light interfering with each other, an optical beat signal corresponding to a Doppler shift amount is detected on the photo diode 150. The optical beat signal detected by the photo diode 150 is frequency-analyzed to calculate the Doppler shift amount, and by this, the blood flow velocity can be calculated.

Incidentally, if the blood flow velocity is measured as described above, it would be better to determine an interval of switching over the emission of the light with the different wavelengths (i.e. a time between when light with one wavelength is emitted and when light with another wavelength is emitted), depending on a physiological phenomenon which is a measurement target, as occasion demands. In accordance with the sampling theorem, a sampling frequency for data collection may be twice or more a cycle of changing the physiological phenomenon which is a measurement target. For example, if the measurement target is limited to the human heartbeat, 8 Hz or more may be used as the sampling frequency because the heartbeat is typically 1Hz, and it is about 4Hz in a faster state. In order to satisfy this condition in the plurality of wavelengths, it is necessary to multiply it by the number of wavelengths used for the measurement. In other words, if a measurement is made using the two different wavelengths as in the embodiment, the switch-over of the light with the different wavelengths may be performed at 16 Hz or more, which is twice 8Hz. As described above, by determining the interval of switching over the emission of the light with the different wavelengths in accordance with the physiological phenomenon which is a measurement target, as occasion demands, it is possible to measure biological information, accurately.

As shown in FIG. 8, the absorbance varies between oxygenated hemoglobin and reduced hemoglobin, and the absorbance also varies depending on the wavelength of the light. Thus, from the measurement using the light with the different wavelengths described above, it is possible to calculate a ratio between oxygenated hemoglobin and reduced hemoglobin, i.e. the blood oxygen saturation level, simultaneously with the calculation of the blood flow velocity. At this time, the wavelengths of the light used for the measurement preferably provide a significant difference in the absorbance between oxygenated hemoglobin and reduced hemoglobin; for example, 700 nm light and 900 nm light may be used. In contrast, light with a wavelength of about 800 nm likely cannot provide an appropriate measurement due to less difference in the absorbance.

Incidentally, if the blood oxygen saturation level is only measured, the change in wavelength by the Doppler shift is not measured, so that the emitted light is not necessarily coherent light, like a laser beam. Thus, the drive current of the two-wavelength laser diode 120 may be controlled to make it function as a light-emitting diode. If it functions as the light-emitting diode, power consumption in the drive is reduced since the drive current is lower than a laser oscillation threshold value. Thus, it is possible to reduce the power consumption, by controlling the drive current of the semiconductor laser depending on the measurement target.

As shown in FIG. 9, for example, in the human subcutaneous structure, an arteriole 410 and a venula 420 are distributed in a dermis tissue. Moreover, blood capillaries 430 branched from the arteriole 410 are distributed toward the vicinity of the epidermis, and their one ends are connected to the venula 420. In connected portions between the arteriole 410 and the blood capillaries 430, there are precapillary sphincters 440 which act like valves and which control the amount of blood flowing into the blood capillaries 430 from the arteriole 410. In other words, it is possible to predict the motion of the precapillary sphincters 440 by simultaneously measuring the blood flow velocity for the blood capillaries 430 and the arteriole 410. Moreover, since the precapillary sphincters 440 are innervated by sympathetic nerves, it is possible to predict a psychological status from the motion of the precapillary sphincters 440. Therefore, by measuring the blood flow velocity for the blood capillaries 430 and the arteriole 410 located in different depths, it is possible to predict the motion of the precapillary sphincters 440 and further predict the psychological status of the measured sample.

As described above, the blood flow velocity varies under an influence of psychological factors; however, it also varies under an influence of breathing. Thus, eliminating the influence of breathing allows more accurate prediction of the psychological status. In contrast, the blood oxygen saturation level, which can be measured simultaneously with the blood flow velocity, is **characterized in that** it varies under the influence of breathing and that it is not influenced by the psychological factors. Thus, by comparing the blood flow velocity and the blood oxygen saturation level, it is possible to eliminate the influence by the variation in breathing from the blood flow velocity and to extract only a component which varies depending on the psychological status. Thus, if a simultaneous measurement can be made between the blood flow velocity and the blood oxygen saturation level, it is possible to predict the psychological status of the measured sample, more highly accurately.

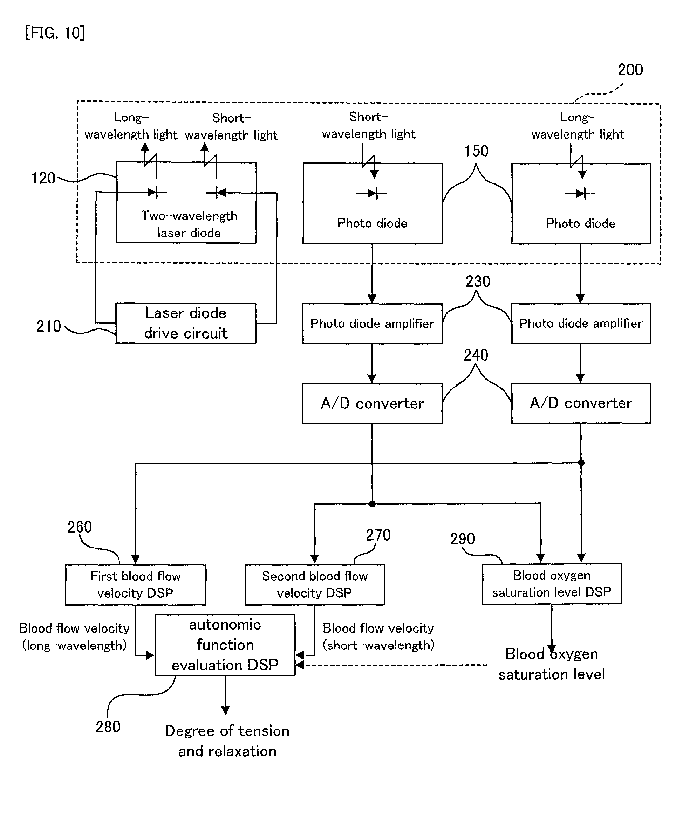

Next, a two-wavelength blood flow sensor apparatus in the second embodiment will be described with reference to FIG. 10. FIG. 10 is a block diagram showing the structure of the two-wavelength blood flow sensor apparatus in the second embodiment. Incidentally, the second embodiment differs from the first embodiment in that the light with the different wavelengths is emitted not sequentially but simultaneously, and its structure is similar. Thus, here, the part different from the first embodiment will be described, and the explanation about the same part will be omitted, as occasion demands.

As shown in FIG. 10, a two-wavelength blood flow sensor apparatus 200 in the second embodiment is provided with the photo diodes 150, each of which corresponds to respective one of the long-wavelength light and the short-wavelength light. Thus, each photo diode 150 is provided with the photo diode amplifier 230 and the A/D converter 240.

In the second embodiment, firstly, signals outputted from the laser diode drive circuit 210 allow the two-wavelength laser diode 120 to simultaneously emit the long-wavelength light and the short-wavelength light. Thus, the first switching circuit 220 (refer to FIG. 6) used in the first embodiment is not necessarily provided.

For the detection of the light reflected or scattered by the measured sample, for example, two photo diodes 150 having an optical band pass filter are used. The optical band pass filter includes a filter which transmits only the long-wavelength light and a filter which transmits only the short-wavelength light. By this, each photo diode detects only the light with either of the wavelengths. Thus, the light with the different wavelengths simultaneously emitted is divided for each wavelength at the time point of the detection by the photo diodes 150. Incidentally, in case of the photo diodes 150 each of which detects only a different predetermined range of wavelength, the optical band pass filter is not necessarily used.

The signals of the light detected are divided by the photo diodes 150 as described above. Thus, it is possible to calculate a result by sending each signal to the first blood flow DSP 260, the second blood flow DSP 270, and the blood oxygen saturation level DSP 290.

In the second embodiment, the simultaneous irradiation of the light with the different wavelengths allows a measurement at the same time point by the two-wavelength light. Thus, it is possible to make an accurate measurement if a parameter which changes in an extremely short time is measured or in similar cases. Moreover, since a process is simultaneously performed on the information obtained from the light with the different wavelengths, it is possible to reduce a time for the measurement.

The present invention can be broadly applied as a blood flow sensor apparatus in a so-called medical engineering field and the like. In particular, the apparatus can be miniaturized small enough to be regularly attached. Thus, taking advantage of the miniaturization, for example, the apparatus is provided for an earphone for hearing aid, a watch, or the like, which allows such applications as always monitoring a health condition for the elderly.

In non-medical fields, the present invention can be applied to home electronics and mobile equipment. For example, by the apparatus being built in a music headphone or the like, it is possible to provide a function of predicting the psychological status of a user and a function of automatically selecting music in accordance with the psychological status. Moreover, by providing the apparatus for a head rest, a steering wheel, or the like in an automobile, the present invention can be applied to data collection or the like to monitor the status of a driver or a fellow passenger and to assist safe driving.

The present invention is not limited to the aforementioned embodiments, but various changes may be made, if desired, without departing from the essence or spirit of the invention which can be read from the claims and the entire specification. A light-emitting sensor apparatus, and a method of detecting biological information using the light-emitting sensor apparatus, all of which involve such changes, are also intended to be within the technical scope of the present invention.

### Industrial Applicability

The light-emitting sensor apparatus and the method of inspecting biological information using the light-emitting sensor apparatus according to the present invention can be applied to, for example, a two-wavelength blood flow sensor apparatus or the like which can measure a blood flow velocity.

## Claims

1. A light-emitting sensor apparatus comprising:
a substrate;
an applying device which is disposed on said substrate and which applies a plurality of light with mutually different wavelengths to a measured sample such that the light overlaps each other at least partially; and
a light receiving device which is disposed on said substrate and which detects light from the measured sample caused by the applied plurality of light, for each wavelength.

2. The light-emitting sensor apparatus according to claim 1, wherein said applying device applies the plurality of light through a common optical system.

3. The light-emitting sensor apparatus according to claim 1, wherein
said applying device sequentially applies the plurality of light, and
said light receiving deice detects the light from the measured sample for each wavelength by time division.

4. The light-emitting sensor apparatus according to claim 1, wherein
said applying device simultaneously applies the plurality of light, and
said light receiving deice has a plurality of light receiving elements which detect the light from the measured sample for each wavelength.

5. The light-emitting sensor apparatus according to claim 1, wherein said applying device and said light receiving device are integrated on said substrate.

6. The light-emitting sensor apparatus according to claim 1, wherein said applying device has a semiconductor laser which generates a plurality of laser beams as the plurality of light.

7. The light-emitting sensor apparatus according to claim 6, wherein the semiconductor laser makes a drive current lower than a laser oscillation threshold value, to thereby function as a light-emitting diode.

8. The light-emitting sensor apparatus according to claim 1, wherein said applying device comprises:
a light source for generating a plurality of source light; and
a reflecting mirror which reflects the generated plurality of source light in a direction of the measured sample so as to be the irradiated plurality of light, at a position irradiated with the generated plurality of source light.

9. The light-emitting sensor apparatus according to claim 8, wherein the reflecting mirror includes, on its surface, at least one ofAu (gold), Cu (copper), Al (aluminum), and Ag (silver).

10. The light-emitting sensor apparatus according to claim 1, further comprising a calculating device for calculating at least one of a blood flow velocity and a blood oxygen saturation level associated with the measured sample on the basis of the detected light.

11. A method of detecting biological information using a light-emitting sensor apparatus comprising: an applying device on a substrate; and a light receiving device, said method comprising:
an applying process of applying a plurality of light with mutually different wavelengths to a measured sample such that the light overlaps each other at least partially; and
a light receiving process of detecting light from the measured sample caused by the applied plurality of light, for each wavelength.
